(19) Europäisches Patentamt
European Patent Office
Office européen des brevets

(11) Veröffentlichungsnummer: **0 275 501 B1**

(12) # EUROPÄISCHE PATENTSCHRIFT

(45) Veröffentlichungstag der Patentschrift: **06.03.91**

(51) Int. Cl.[5]: **C07C 63/66**, C07C 63/74, C07C 65/28, C07C 51/353, C07C 205/06, C07C 255/50

(21) Anmeldenummer: **87118718.3**

(22) Anmeldetag: **17.12.87**

(54) **Verfahren zur Herstellung von (Z)-2-(2-Arylethenyl)-arylcarbonsäuren.**

(30) Priorität: **24.12.86 DE 3644463**

(43) Veröffentlichungstag der Anmeldung:
**27.07.88 Patentblatt 88/30**

(45) Bekanntmachung des Hinweises auf die Patenterteilung:
**06.03.91 Patentblatt 91/10**

(84) Benannte Vertragsstaaten:
**AT BE CH DE ES FR GB GR IT LI NL SE**

(56) Entgegenhaltungen:
**DE-A- 2 039 993**
**DE-A- 3 524 199**

**INDIAN JOURNAL OF CHEMISTRY, Band 22B, September 1983, Seiten 850-852; N.S. NARASIMHAN et al.: "Synthetic application of lithiation reaction: Part XVIII - Synthesis of ( + -)-3,4-dihydro-3-phenylisocoumarin & aglycone of ( + -)-dihydrohomalicine"**

(73) Patentinhaber: **BASF Aktiengesellschaft**
**Carl-Bosch-Strasse 38**
**W-6700 Ludwigshafen(DE)**

(72) Erfinder: **Thyes, Marco, Dr.**
**Thorwaldsenstrasse 1**
**W-6700 Ludwigshafen(DE)**
Erfinder: **Steiner, Gerd, Dr.**
**Oberer Waldweg 1**
**W-6719 Kirchheim(DE)**

**EP 0 275 501 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein verbessertes Verfahren zur Herstellung von (Z)-2-(2-Arylethenyl)-arylcarbonsäuren der allgemeinen Formel I

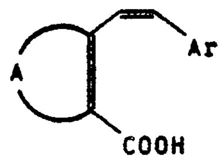

(I)

in welcher A eine Gruppierung zur Vervollständigung eines aromatischen Ringsystems und Ar einen aromatischen Rest bedeutet, durch Umsetzung einer 2-Formyl-arylcarbonsäure der allgemeinen Formel II

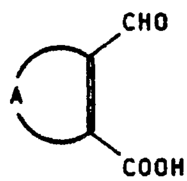

(II)

mit einem (Arylmethyl)phosphoniumsalz der allgemeinen Formel III,

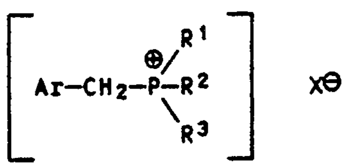

(III)

in der $R^1$, $R^2$ und $R^3$ für organische Reste stehen und X Halogen bedeutet, in Gegenwart einer Base bei Temperaturen zwischen -20 °C und +30 °C, welches dadurch gekennzeichnet ist, daß man als Reaktionsmedium ein $C_1$-$C_5$-Alkanol.

Aus der DE-OS 16 18 706 ist bekannt, trifluormethylsubstituierte Phthalaldehydsäuren mit Benzyltriphenylphosphoniumchlorid in Tetrahydrofuran bei Raumtemperatur (20 bis 25 °C) zu den entsprechenden 2-(2-Arylethenyl)-benzoesäurederivaten I′ umzusetzen und diese Verbindungen in die 7-Ringketone IV′

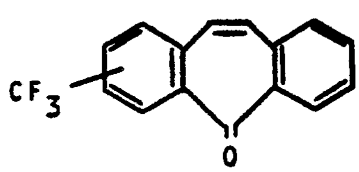

(IV')

zu überführen.

Da bei dieser Wittig-Reaktion jedoch überwiegend die E-Isomeren entstehen, die aus sterischen Gründen für die Cyclisierung zu den 7-Ringketonen von vornherein ungeeignet sind, mußten die Verbindungen I′ vor dem Ringschluß zu den Verbindungen I″

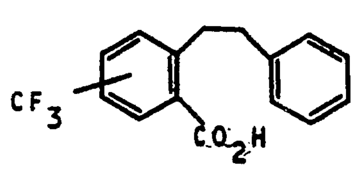

(I")

hydriert und nach dem Ringschluß zu den Verbindungen IV′ dehydriert werden.

Auch nach dem Verfahren aus dem Ind.J.Chem., Vol. 22B (1983) 850 erhält man fast ausschließlich die E-Isomeren.

Allgemein lag der Erfindung die Aufgabe zugrunde, die 7-Ringketone IV

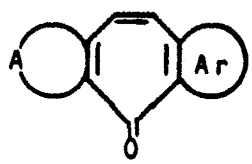

(IV)

auf einfachere Weise zugänglich zu machen, wobei es die spezielle Aufgabe war, die Wittig-Reaktion so zu gestalten, daß man hierbei größtenteils die Z-Isomeren I erhält, die unmittelbar der Cyclisierung zu den Verbindungen IV zugänglich sind.

Demgemäß wurde ein Verfahren zur Herstellung von (Z)-2-(2-Arylethenyl)-arylcarbonsäuren der allgemeinen Formel I,

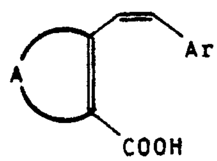

(I)

in welcher A eine Gruppierung zur Vervollständigung eines aromatischen Ringsystems und Ar einen aromatischen Rest bedeutet, durch Umsetzung einer 2-Formyl-arylcarbonsäure der allgemeinen Formel II

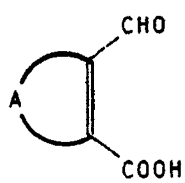

(II)

mit einem (Arylmethyl)phosphoniumsalz der allgemeinen Formel 117.

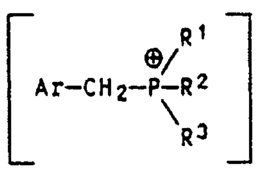

X⊖ (III)

in der $R^1$, $R^2$ und $R^3$ fur organische Reste stehen und X Halogen bedeutet. in Gegenwart einer Base bei Temperaturen zwischen (-20) und +30°C gefunden, welches dadurch gekennzeichnet ist, daß man die Umsetzung in Gegenwart eines $C_1$-$C_5$-Alkanols als alleinigem Lösungsmittel ausführt.

Als $C_1$-$C_5$-Alkanole eignen sich Methanol, Ethanol, n-Propanol, iso-Propanol, n-Butanol, iso-Butanol, sec.-Butanol, tert.-Butanol, die Pentanole wie n-Pentanol oder deren Gemische. Besonders bevorzugt wird in Methanol, Ethanol oder tert.-Butanol gearbeitet.

Die Menge des $C_1$-$C_5$-Alkanols beträgt vorzugsweise 0,8 bis 50 ml/g II, besonders 1 bis 10 ml/g II.

Die zur Durchführung des erfindungsgemäßen Verfahrens benotigten (Arylmethyl)phosphoniumsalze III sind entweder bekannt oder können nach bekannten Methoden (Houben-Weyl, Methoden der Organischen Chemie, Bd. 1211. Seite 79ff) hergestellt werden. Ebenso sind die Verbindungen II entweder bekannt oder nach bekannten Methoden herstellbar.

Als Reste $R^1$ bis $R^3$ des Phosphoniumkations

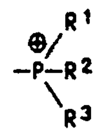

sind solche geeignet, die mit dem Phosphoniumsalz nicht reagieren wie Arylgruppen, beispielsweise Phenyl, substituierte Phenylreste, wie z.B. Tolyl, Xylyl, Mesityl oder Methoxyphenyl, oder auch mehrkernige Reste, wie z.B. Naphthyl, Anthryl oder Phenanthryl. Vorzugsweise bedeuten die Reste $R^1$ bis $R^3$ Phenyl. Als Gegenion $X^{\ominus}$ eignen sich Halogene, vorzugsweise Chlor und Brom.

Man nimmt die Umsetzung bei (-20) bis +30 ° C, vorzugsweise bei (-10) bis +10 ° C vor, wobei es sich in aller Regel empfiehlt, bei Normaldruck zu arbeiten.

Der Strukturteil

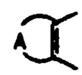

in den Verbindungen I bzw. II entspricht vorzugsweise dem Benzol, dem 1,2- und 2,3-Naphthalin sowie den Halogen-, $C_1$-$C_4$-Alkyl-, $C_1$-$C_4$-Alkoxy- und Trihalogenalkylderivaten dieser Stammkörper.

Ar bedeutet beispielsweise

- unsubstituierte Aromaten wie Phenyl, 1-Naphthyl, 2-Naphthyl und Anthryl, besonders Phenyl,
- durch Halogen substituiertes Phenyl wie Monohalogenphenyl, Dihalogenphenyl und Trihalogenphenyl, besonders Monohalogenphenyl wie 2-, 3-, 4-Fluorphenyl, 2-, 3-, 4-Chlorphenyl, 2-, 3-, 4-Bromphenyl, 4-Jodphenyl und Dihalogenphenyl wie 2,3-, 2,4-, 2,5-, 3,4-, 3,5-Difluorphenyl, 2,3-, 2,4-, 2,5-, 3,4-, 3,5-Dichlorphenyl, 2,3-, 2,4-, 2,5-, 3,4-, 3,5-Dibromphenyl, 2-Chlor-4-fluorphenyl, 3-Brom-5-chlorphenyl und 2-Fluor-4-bromphenyl,
- durch $C_1$-$C_4$-Alkyl substituiertes Phenyl wie Mono-$C_1$-$C_4$-alkylphenyl, Di-$C_1$-$C_4$-alkylphenyl und Tri-$C_1$-$C_4$-alkylphenyl, besonders Mono-$C_1$-$C_4$-alkylphenyl wie 2-, 3-, 4-Methylphenyl, 2-, 3-, 4-Ethylphenyl, 2-, 3-, 4-n-Propylphenyl, 2-, 3-, 4-iso-Propylphenyl, 2-, 3-, 4-n-Butylphenyl, 2-, 3-, 4-iso-Butylphenyl, 2-, 3-, 4-sec.-Butylphenyl, 2-, 3-, 4-tert.-Butylphenyl und Di-$C_1$-$C_4$-alkylphenyl wie 2,3-, 2,4-, 2,5-, 3,4-, 3,5-Dimethylphenyl, 2,3-, 2,4-, 2,5-, 3,4-, 3,5-Diethylphenyl, 2,3-, 2,4-, 2,5-, 3,4-, 3,5-Di-n-propylphenyl, 2,3-, 2,4-, 2,5-, 3,4-, 3,5-Di-isopropylphenyl, 2,3-, 2,4-, 2,5-, 3,4-, 3,5-Di-n-butylphenyl, 2,3-, 2,4-, 2,5-, 3,4-, 3,5-Di-iso-butylphenyl, 2,3-, 2,4-, 2,5-, 3,4-, 3,5-Di-sec.-butylphenyl, 2,3-, 2,4-, 2,5-, 3,4-, 3,5-Di-tert.-butylphenyl, 2-Methyl-4-tert.-butylphenyl, 3-Methyl-5-ethylphenyl und 3-Ethyl-4-n-propylphenyl,
- durch $C_1$-$C_4$-Alkoxy substituiertes Phenyl wie Mono-$C_1$-$C_4$-alkoxyphenyl, Di-$C_1$-$C_4$-alkoxyphenyl und Tri-$C_1$-$C_4$-alkoxyphenyl, besonders Mono-$C_1$-$C_4$-alkoxyphenyl wie 2-, 3-, 4-Methoxyphenyl, 2-, 3-, 4-Ethoxyphenyl, 2-, 3-, 4-n-Propoxyphenyl, 2-, 3-, 4-iso-Propoxyphenyl, 2-, 3-, 4-n-Butoxyphenyl, 2-, 3-, 4-iso-Butoxyphenyl, 2-, 3-, 4-sec.-Butoxyphenyl, 2-, 3-, 4-tert.-Butoxyphenyl und Di-$C_1$-$C_4$-alkoxyphenyl wie 2,3-, 2,4-, 2,5-, 3,4-, 3,5-Dimethoxyphenyl, 2,3-, 2,4-, 2,5-, 3,4-, 3,5-Diethoxyphenyl, 2,3-, 2,4-, 2,5-, 3,4-, 3,5-Di-n-propoxyphenyl, 2,3-, 2,4-, 2,5-, 3,4-, 3,5-Di-iso-propoxyphenyl, 2,3-, 2,4-, 2,5-, 3,4-, 3,5-Di-n-butoxyphenyl, 2,3-, 2,4-, 2,5-, 3,4-, 3,5-Di-iso-butoxyphenyl, 2,3-, 2,4-, 2,5-, 3,4-, 3,5-Di-sec.-butoxyphenyl, 2,3-, 2,4-, 2,5-, 3,4-, 3,5-Di-tert.-butoxyphenyl, 2-Methoxy-4-tert.-butoxyphenyl und 3-Ethoxy-5-n-propoxyphenyl,
- durch Trihalogenmethyl substituiertes Phenyl wie Mono-, Di- und Trihalogenmethylphenyl, besonders Monotrichlormethylphenyl wie 2-, 3-, 4-Trichlormethylphenyl, Monotrifluormethylphenyl wie 2-, 3-, 4-Trifluormethylphenyl, Di-trichlormethylphenyl wie 2,3-, 2,4-, 2,5-, 3,4-, 3,5-Di-trichlormethylphenyl und Di-trifluormethylphenyl wie 2,3-, 2,4-, 2,5-, 3,4-, 3,5-Di-trifluormethylphenyl,
- durch Cyan substituiertes Phenyl, wie Monocyanphenyl, Dicyanphenyl, Tricyanphenyl, besonders Monocyanphenyl, wie 2-Cyanphenyl, 3-Cyanphenyl, 4-Cyanphenyl, und Dicyanphenyl, wie 2,3-Dicyanphenyl, 2,4-Dicyanphenyl, 2,5-Dicyanphenyl, 3,4-Dicyanphenyl, 3,5-Dicyanphenyl,
- durch Nitro substituiertes Phenyl, wie Mononitrophenyl, Dinitrophenyl, besonders Mononitrophenyl, wie 2-Nitrophenyl, 3-Nitrophenyl, 4-Nitrophenyl,
- durch $C_1$-$C_4$-Alkylmercapto substituiertes Phenyl wie Mono-$C_1$-$C_4$-Alkylmercaptophenyl, Di-$C_1$-$C_4$-Alkylmercaptophenyl, Tri-$C_1$-$C_4$-Alkylmercaptophenyl, besonders Mono-$C_1$-$C_4$-Alkylmercaptophenyl, wie 2-, 3-, 4-Methylmercaptophenyl, 2-, 3-, 4-Ethylmercaptophenyl, 2-, 3-, 4-n-Propylmercaptophenyl,

2-, 3-, 4-Isopropylmercaptophenyl, 2-, 3-, 4-n-Butylmercaptophenyl.

Besonders bevorzugt sind solche Reste Ar, in denen eine ortho-Position zur Ethenylgruppierung mit Wasserstoff besetzt ist.

Zur Herstellung der (Z)-2-(2-Arylethenyl)-arylcarbonsäuren I kann man sowohl die in situ hergestellten als auch die isolierten (Arylmethyl)phosphoniumsalze III einsetzen. Die Umsetzung zu den Verbindungen I erfolgt durch Reaktion einer 2-Formyl-arylcarbonsäure II mit einer Verbindung III unter Verwendung von bevorzugt mindestens äquimolaren Mengen III in Gegenwart einer Base. Besonders bevorzugt werden äquimolare Mengen der Verbindungen II und III. Zweckmäßigerweise sollte man das Arylmethylphosphoniumsalz III vorlegen und zum Schluß die Base zufügen. Je Mol II werden mindestens 2 Mol einer Base eingesetzt. Bevorzugt wird mit 2,1 bis 2,5 Mol Base pro Mol II gearbeitet.

Als Basen kann man Alkali- oder Erdalkalimetallsalze der $C_1$-$C_5$-Alkanole oder Alkali- und Erdalkalimetallcarbonate verwenden, besonders bevorzugt werden Natriummethylat und Alkalimetallcarbonate.

Wie aus Agric.Biol.Chem. 45, (1981) 1669 bekannt ist, werden einige Verbindungen I als Pflanzenwachstumsregulatoren empfohlen. Weiterhin können aus den Z-Isomeren in isolierter Form oder eingesetzt als E/Z-Isomerengemisch tricyclische Ketone hergestellt werden, die zu pharmakologisch wirksamen tricyclischen Verbindungen umgesetzt werden können (DE-OS 30 09 034).

Unter den Verbindungen I, die nach diesem Verfahren erhältlich sind, sind die folgenden Z-Isomeren bevorzugt:

2-(2-Phenylethenyl)benzoesäure
2-[2-(2-Chlorphenyl)ethenyl]benzoesäure
2-[2-(3-Chlorphenyl)ethenyl]benzoesäure
2-[2-(4-Chlorphenyl)ethenyl]benzoesäure
2-[2-(3,4-Dichlorphenyl)ethenyl]benzoesäure
2-[2-(3-Fluorphenyl)ethenyl]benzoesäure
2-[2-(3-Trifluormethyl-phenyl)ethenyl]benzoesäure
2-[2-(3-Methoxyphenyl)ethenyl]benzoesäure
2-[2-(2-Bromphenyl)ethenyl]benzoesäure
2-[2-(3-Bromphenyl)ethenyl]benzoesäure
2-[2-(4-Bromphenyl)ethenyl]benzoesäure
2-[2-(2-Fluorphenyl)ethenyl]benzoesäure
2-[2-(4-Fluorphenyl)ethenyl]benzoesäure
2-[2-(2,3-Dichlorphenyl)ethenyl]benzoesäure
2-[2-(2,5-Dichlorphenyl)ethenyl]benzoesäure
2-[2-(3,5-Dichlorphenyl)ethenyl]benzoesäure
2-[2-(2,3-Dibromphenyl)ethenyl]benzoesäure
2-[2-(3,4-Dibromphenyl)ethenyl]benzoesäure
2-[2-(2-Tolyl)ethenyl]benzoesäure
2-[2-(3-Tolyl)ethenyl]benzoesäure
2-[2-(4-Tolyl)ethenyl]benzoesäure
2-[2-(2-Ethylphenyl)ethenyl]benzoesäure
2-[2-(3-Ethylphenyl)ethenyl]benzoesäure
2-[2-(4-Ethylphenyl)ethenyl]benzoesäure
2-[2-(3-Propylphenyl)ethenyl]benzoesäure
2-[2-(3-Isopropylphenyl)ethenyl]benzoesäure
2-[2-(3-Butylphenyl)ethenyl]benzoesäure
2-[2-(2,3-Dimethylphenyl)ethenyl]benzoesäure
2-[2-(3,4-Dimethylphenyl)ethenyl]benzoesäure
2-[2-(3,5-Dimethylphenyl)ethenyl]benzoesäure
2-[2-(2-Methoxyphenyl)ethenyl]benzoesäure
2-[2-(4-Methoxyphenyl)ethenyl]benzoesäure
2-[2-(2-Ethoxyphenyl)ethenyl]benzoesäure
2-[2-(3-Ethoxyphenyl)ethenyl]benzoesäure
2-[2-(4-Ethoxyphenyl)ethenyl]benzoesäure
2-[2-(2,3-Dimethoxyphenyl)ethenyl]benzoesäure
2-[2-(3,4-Dimethoxyphenyl)ethenyl]benzoesäure
2-[2-(2,5-Dimethoxyphenyl)ethenyl]benzoesäure
2-[2-(2-Cyanphenyl)ethenyl]benzoesäure
2-[2-(3-Cyanphenyl)ethenyl]benzoesäure

2-[2-(4-Cyanphenyl)ethenyl]benzoesäure
2-[2-(2-Nitrophenyl)ethenyl]benzoesäure
2-[2-(3-Nitrophenyl)ethenyl]benzoesäure
2-[2-(4-Nitrophenyl)ethenyl]benzoesäure
2-[2-[2-(Methylmercapto)phenyl]ethenyl]benzoesäure
2-[2-[3-(Methylmercapto)phenyl]ethenyl]benzoesäure
2-[2-[4-(Methylmercapto)phenyl]ethenyl]benzoesäure
2-[2-[2-(Ethylmercapto)phenyl]ethenyl]benzoesäure
2-[2-[3-(Ethylmercapto)phenyl]ethenyl]benzoesäure
2-[2-[4-(Ethylmercapto)phenyl]ethenyl]benzoesäure

Die Verhältnisse von E- und Z-Isomerenanteil in den Gemischen konnte jeweils durch $^{13}C$-NMR-Spektroskopie bestimmt werden.

Beispiel 1

Herstellung von (E/Z)-2-[2-(3-Chlorphenyl)ethenyl]benzoesäure

Zu 260 g (991 mmol) Triphenylphosphin in 150 ml Methanol wurden unter Rühren 160 g (994 mmol) 3-Chlorbenzylchlorid zugetropft und 2 Stunden unter Rückfluß erhitzt. Die auf 0° C abgekühlte Reaktionsmischung wurde unter Rühren innerhalb ca. 1 min mit 150 g (999 mmol) o-Phthalaldehydsäure versetzt. Anschließend wurden bei 0° C innerhalb ca. 45 min 450 g (2,5 mol) einer 30 %igen Natriummethylat-Lösung in Methanol zugetropft. Nach beendigtem Zutropfen wurde noch 3 h bei 0° C nachgerührt und das Reaktionsgemisch dann auf ein gerührtes Gemisch aus 1,5 kg Eis und 3,5 l Wasser gegossen. Es wurde abgesaugt und der Filterrückstand (Triphenylphosphinoxid) mit ca. 700 ml Wasser gewaschen. Das Waschwasser wurde zu der Mutterlauge gegeben. Die vereinigten wäßrigen Phasen wurden zwecks Entfernung des darin noch enthaltenen Triphenylphosphinoxids mit insgesamt 1 l Methylenchlorid extrahiert und dann mit ca. 150 ml (ca. 1,87 mol) konzentrierter Salzsäure stark sauer gestellt. Der dabei gebildete Niederschlag wurde abgesaugt, gut mit Wasser gewaschen und im Vakuumtrockenschrank getrocknet. Man erhielt 232,3 g (91 %) (E/Z)-2-[2-(3-Chlorphenyl)ethenyl]benzoesäure im Verhältnis von ~ 23:77 als farblose Kristalle vom Schmelzpunkt 118 bis 121° C.

Beispiel 2

Herstellung von (E/Z)-2-[2-(3-Chlorphenyl)ethenyl]benzoesäure

Zu 41,2 g (157 mmol) Triphenylphosphin in 25 ml Methanol wurden unter Rühren 25,2 g (156 mmol) 3-Chlorbenzylchlorid zugetropft und 2 Stunden unter Rückfluß erhitzt. Die auf Raumtemperatur abgekühlte Reaktionsmischung wurde unter Rühren nacheinander mit 23,6 g (157 mmol) o-Phthalaldehydsäure und 47,9 g (347 mmol) Kaliumcarbonat versetzt. Das Reaktionsgemisch wurde noch 7 Tage bei Raumtemperatur nachgerührt und wie üblich aufgearbeitet. Man erhielt 32,0 g (79 %) (E/Z)-2-[2-(3-Chlorphenyl)ethenyl]-benzoesäure im Verhältnis von ~ 31:69 als farblose Kristalle vom Schmelzpunkt 113 bis 123° C.

Beispiel 3

Herstellung von (E/Z)-2-[2-(2-Chlorphenyl)ethenyl] benzoesäure

Zu 78 g (297 mmol) Triphenylphosphin in 150 ml Methanol wurden unter Rühren 48,3 g (300 mmol) 2-Chlorbenzylchlorid zugetropft und 2 Stunden unter Rückfluß erhitzt. Die auf 0° C abgekühlte Reaktionsmischung wurde unter Rühren innerhalb ca. 1 min mit 46.4 g (309 mmol) o-Phthalaldehydsäure versetzt. Danach wurden bei 0 bis 10° C unter Rühren innerhalb 45 min 135 g (750 mmol) einer 30 %igen Natriummethylat-Lösung in Methanol zugetropft, 3 Stunden bei 0 bis 10° C nachgerührt und wie üblich aufgearbeitet. Man erhielt 72 g (94%) (E/Z)-2-[2-(2-Chlorphenyl) ethenyll-benzoesäure im Verhältnis von ~

11:89 als farblose Kristalle vom Schmelzpunkt 144 bis 148 °C.

Beispiel 4

Herstellung von (E/Z)-2-[2-(4-Chlorphenyl)ethenyl]benzoesäure

Analog Beispiel 3 wurden 48,3 g (300 mmol) 4-Chlorbenzylchlorid umgesetzt, wobei man 67,0 g (87 %) (E/Z)-2-[2-(4-Chlorphenyl) ethenyl] benzoesäure im Verhältnis von ~ 32:68 als farblose Kristalle vom Schmelzpunkt 127 bis 131 °C erhielt.

Beispiel 5

Herstellung von (E/Z)-2-[2-(3-Fluorphenyl)ethenyl]benzoesäure

Zu 86 g (328 mmol) Triphenylphosphin in 150 ml Methanol wurden unter Rühren 47.6 g (329 mmol) 3-Fluorbenzylchlorid zugetropft und 2 Stunden unter Rückfluß erhitzt. Die auf 0 °C abgekühlte Reaktionsmischung wurde unter Rühren innerhalb ca. 1 min mit 50,5 g (336 mmol) o-Phthalaldehydsäure versetzt. Danach wurden bei -5 bis 0 °C unter Rühren innerhalb 45 min 149,4 g (830 mmol) einer 30 %igen Natriummethylat-Lösung in Methanol zugetropft, 3 Stunden bei 0 °C nachgerührt und wie üblich aufgearbeitet. Man erhielt 60,0 g (76 %) (E/Z)-2-[2-(3-Fluorphenyl)ethenyl]benzoesäure im Verhältnis von ~ 27:73 als farblose Kristalle vom Schmelzpunkt 105 bis 118 °C.

Weitere hergestellte Verbindungen I, in denen A jeweils -CH=CH-CH=CH- bedeutet, sind in der folgenden Tabelle zusammengestellt.

| Ar | Base | hergestellt nach Bsp.Nr. | Reaktionstemp. [°C] | Schmp. [°C] | Ausbeute [%] | Z/E-Verhältnis |
|---|---|---|---|---|---|---|
| Phenyl | 30%ige $NaOCH_3$ in $CH_3OH$ | 1 | 0-5 | 120-123 | 77 | ~ 58/42 |
| 3,4-Dichlorphenyl | " | 1 | 0 | 151-158 | 84 | ~ 80/20 |
| 3-(Trifluormethyl)phenyl | " | 1 | (-5)-0 | 127-132 | 79 | ~ 84/16 |
| 3-Methoxyphenyl | " | 1 | 0 | 101-105 | 72 | ~ 61/39 |
| 3-Cyanphenyl | " | 1 | 0 | 170-182 | 84 | ~ 87/13 |
| 3-Nitrophenyl | " | 1 | 0 | 165-170 | 83 | ~ 86/14 |

## Ansprüche

1. Verfahren zur Herstellung von (Z)-2-(2-Arylethenyl)-arylcarbonsäuren der allgemeinen Formel I

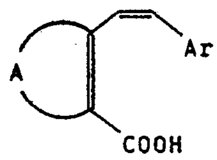

(I)

in welcher A eine Gruppierung zur Vervollständigung eines aromatischen Ringsystems und Ar einen aromatischen Rest bedeutet, durch Umsetzung einer 2-Formyl-arylcarbonsäure der allgemeinen Formel II

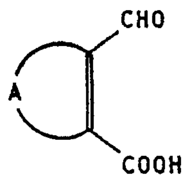

(II)

mit einem (Arylmethyl)phosphoniumsalz der allgemeinen Formel III,

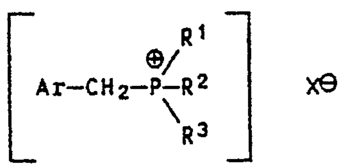

(III)

in der $R^1$, $R^2$ und $R^3$ für organische Reste stehen und X Halogen bedeutet, in Gegenwart einer Base bei Temperaturen zwischen (-20) und +30 °C, dadurch gekennzeichnet, daß man die Umsetzung in Gegenwart eines $C_1$-$C_5$-Alkanols und/oder eines $C_2$-$C_4$-Alkandiols ausführt.

2. Verfahren nach Anspruch 1, dadurch gekennzeichnet, daß man als 2-Formyl-arylcarbonsäure II Phthaldehydsäure IIa

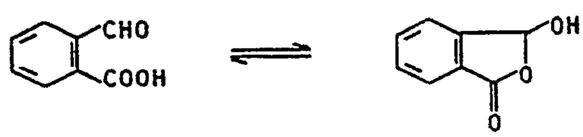

(IIa)

verwendet.

3. Verfahren nach den Ansprüchen 1 und 2. dadurch gekennzeichnet, daß man als (Arylmethyl) phosphoniumsalz III (Arylmethyl) triphenylphosphoniumchlorid oder -bromid verwendet.

4. Verfahren nach den Ansprüchen 1 bis 3, dadurch gekennzeichnet, daß man 0,8 bis 50 ml $C_1$-$C_5$-Alkanol/g II verwendet.

## Claims

1. A process for preparing a (Z)-2-(2-arylethenyl)-aryl-carboxylic acid of the general formula I

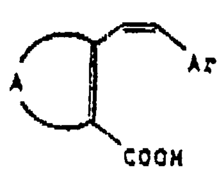

(I)

where A is a group for completing an aromatic ring system and Ar is an aromatic radical, by reacting a 2-formylarylcarboxylic acid of the general formula II

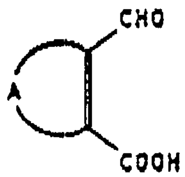

(II)

with an (arylmethyl)phosphonium salt of the general formula III

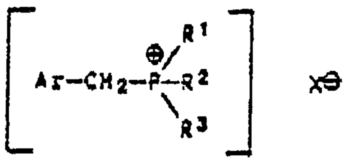

(III)

where $R^1$, $R^2$ and $R^3$ are each organic radicals and x is halogen, in the presence of a base at from (-20) to +30 °C, which comprises carrying out the reaction in the presence of a $C_1$-$C_5$-alkanol as sole solvent.

2. A process as claimed in claim 1, wherein the 2-formylarylcaboxylic acid II is a phthalaldehydic acid IIa

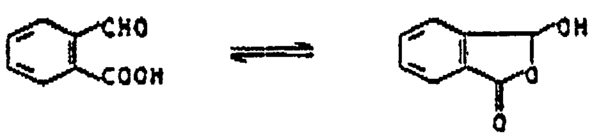

(IIa)

3. A process as claimed in claim 1 or 2, wherein the (arylmethyl)phosphonium salt III is an [arylmethyl)-triphenylphosphonium chloride or bromide.

4. A process as claimed in claim 1 or 2 or 3, wherein from 0.8 to 50 ml of $C_1$-$C_5$-alkanol/g of II are used.

## Revendications

1. Procédé de préparation des acides (Z)-2-(2-aryléthényl) -arylcarboxyliques de formule générale I

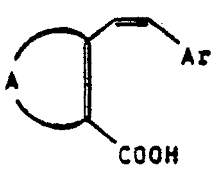

(I)

dans laquelle A représente un groupement complétant un système cyclique aromatique et Ar représente un radical aromatique, par réaction d'un acide 2-formyl-arylcarboxylique de formule générale II

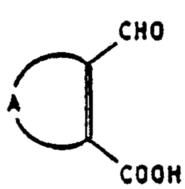

(II)

avec un sel d' (arylméthyl) -phosphonium de formule générale III

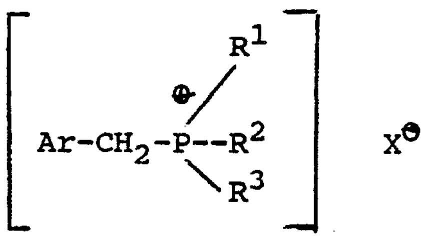

(III)

dans laquelle $R^1$, $R^2$ et $R^3$ représentent des radicaux organiques et X un halogène, en présence d'une base, à des températures allant de -20 à +30 degrés C, caractérisé en ce que l'on effectue la réaction en présence d'un solvant unique consistant en un alcanol en C1-C5.

**2.** Procédé selon la revendication 1, caractérisé en ce que l'on utilise en tant qu'acide 2-formylarylcarboxylique II, l'acide phtalaldéhydique IIa

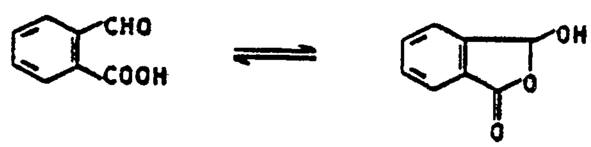

(IIa)

**3.** Procédé selon les revendications 1 et 2, caractérisé en ce que l'on utilise en tant que sel d'-(arylméthyl)phosphonium III le chlorure ou le bromure d' (arylméthyl) -triphénylphosphonium.

**4.** Procédé selon les revendications 1 à 3, caractérisé en ce que l'on utilise de 0,8 à 50 ml d'alcanol en C 1C 5 par g du composé II.